# EUROPEAN PATENT SPECIFICATION

(11) **EP 1 250 893 B1**
(45) Date of publication and mention of the grant of the patent: **31.10.2007**
(21) Application number: 02252310.4
(22) Date of filing: 28.03.2002
(51) Int. Cl.: A61B 18/22, A61C 1/00

(54) **Hand apparatus for light delivery**
Lichtlieferungshandgerät
Appareil à main d'apport de lumière

(30) Priority: 29.03.2001 GB 0107853
(43) Date of publication of application: 23.10.2002
(73) Proprietor: Carl Zeiss Meditec AG, 07745 Jena (DE)
(72) Inventor: Silk, Graham David, Perthshire KY13ONX (GB); Thomas, Richard Jolyon, Clackmannanshire FK147LX (GB); Lynch, Bruce Robert, Glasgow, Scotland G12 OSE (GB)
(74) Representative: Murnane, Graham John

(56) References cited:
- EP-A- 0 882 432
- US-A- 4 744 624
- US-A- 4 884 568
- US-A- 4 895 145
- US-A- 5 300 061

## Description

This invention relates to a hand apparatus for delivery of a beam of light, for example a collimated laser beam, to a point of application. In particular the invention relates to a hand apparatus for delivery of a laser beam to a point of application on a tooth in a patient's mouth.

Hand pieces are used in medicine, dentistry and other forms of treatment to apply light locally to an area being treated. The light may be generated at a base unit and transmitted along a cable to a fixed hand piece. Alternatively the base unit may include a power source, with electrical power transmitted along a cable to a hand piece to generate the light at the hand piece itself. The flexible cable connection allows the operator of the hand piece to move the hand piece easily to the required point of application.

The known hand pieces suffer from the disadvantage that they must be sterilised between uses. The hand pieces contain precise optical components, so that they cannot be sterilised in the way that mechanical apparatus can. One solution is to provide a disposable sleeve around the hand piece. The sleeve is discarded and replaced between uses of the hand piece. However this solution suffers from the disadvantage that the sleeve cannot be totally effective in eliminating contamination from one use to the next.

US 4 744 624 discloses a handheld apparatus which permits use on a once-and-for-all basis.

It is an object of the present invention to provide a hand apparatus for delivery of a beam of light, for example a collimated laser beam, to a point of application, wherein the apparatus is inexpensive to manufacture and simple to connect to a light source, so that the hand apparatus can be used as a single use apparatus, eliminating the requirement to sterilise the apparatus for reuse.

According to a first aspect of the present invention there is provided a hand apparatus comprising a housing, a light source, an optical connector, an elongate sleeve having a light emitter at one end, and an optical carrier extending from the optical connector to the light emitter, characterised in that:
the optical connector comprises a first optical connector (22) which is connectable to the light source (3) via a number of optical fibres and a second optical connector (20) which is connectable to the first optical connector (22) and the optical carrier (40),
and that the second optical connector (20) is provided in the housing (10).

Preferably the optical connector is a male or female optical connector adapted for connection of optical telecommunication fibres. Preferably the optical connector includes a resiliently biased optical coupler to ensure contact between the optical coupler of the connector and the light source to which it is to be connected.

The apparatus may include a safety means which prevents light being transmitted if the optical connector is disconnected from the light source. Preferably the apparatus includes a switch adapted to operate a detent member which allows disconnection of the connector from the light source upon operation of the switch.

The light emitter may be an isotip emitter of the sort described in United States Patent No. 5,073,402.

Preferably the housing is of moulded plastic. The housing may be provided in two snap-fit parts. Preferably the housing is an elongate housing and includes means for retaining the optical carrier within the housing. Preferably the retaining means is adapted to permit longitudinal movement of the optical carrier relative to the channel during connection of the connector to a light source.

Preferably the retaining means includes an internal channel extending from the connector to the sleeve. Preferably the optical carrier extends along the channel.

Preferably the optical carrier includes at least one optical fibre.

The apparatus may also comprise a fluid delivery system. Preferably the fluid delivery system includes a reservoir, a fluid outlet adjacent the light emitter, and a conduit connecting the reservoir and the fluid outlet. The conduit may allow the passage of fluid from the fluid reservoir to the outlet adjacent the light emitter via the housing and the sleeve.

Preferably the sleeve is a resiliently flexible sleeve. The sleeve may be plastically deformable such that an operator can deform the sleeve to a required shape. Preferably the sleeve is adapted to permit longitudinal movement of the optical carrier relative to the sleeve upon deformation or flexing of the sleeve. Preferably the sleeve is of stainless steel.

Preferably the first optical connector includes a first optical coupler and the second optical connector includes a second resiliently biased optical coupler to ensure contact between the first and second optical couplers when the first and second optical connectors are connected.

The light delivery system may include a safety means which prevents light being transmitted if the optical connector is disconnected from the light source. The safety means may be provided on the light source or the hand apparatus. Preferably the light delivery system includes a switch adapted to operate a detent member which allows disconnection of the first and second optical connectors upon operation of the switch.

The hand apparatus further comprises a housing, an elongate sleeve having a light emitter at one end, and an optical carrier extending from the second optical connector to the light emitter.

The light delivery system may also comprise a fluid delivery system. Preferably the fluid delivery system includes a reservoir, a fluid outlet adjacent the light emitter, and a conduit connecting the reservoir and the fluid outlet. The conduit may allow the passage of fluid from the fluid reservoir to the outlet adjacent the light emitter via the housing and the sleeve. An embodiment of the invention will now be described, by way of example only, with reference to the accompanying figures, where:
Fig. 1 is a cross sectional view of a hand apparatus according to a first embodiment of the invention;
Fig. 2 is an isometric cross sectional view of the hand apparatus of Fig. 1;
Fig. 3 is a partial cross sectional view at III-III of the hand apparatus of Fig. 1;
Fig. 4 is an isometric view of the hand apparatus of Fig. 1; and
Fig. 5 is a partial enlarged view of the switch of the hand apparatus of Fig. 1.

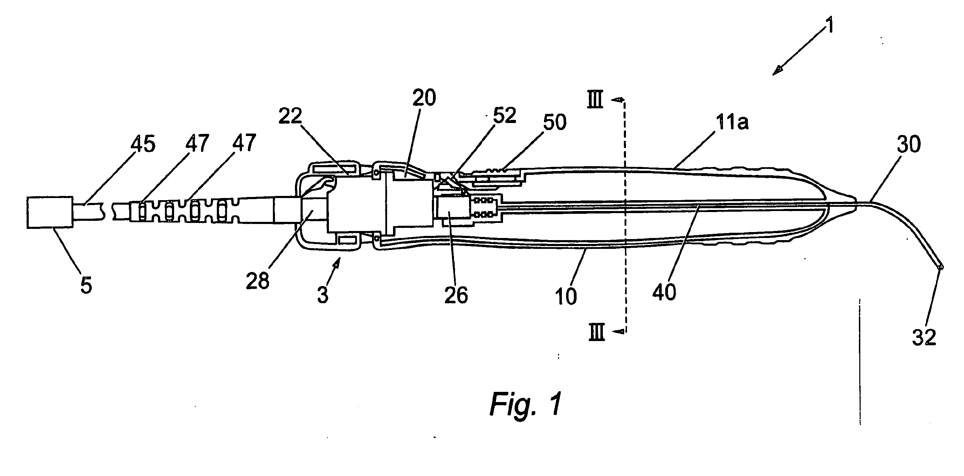

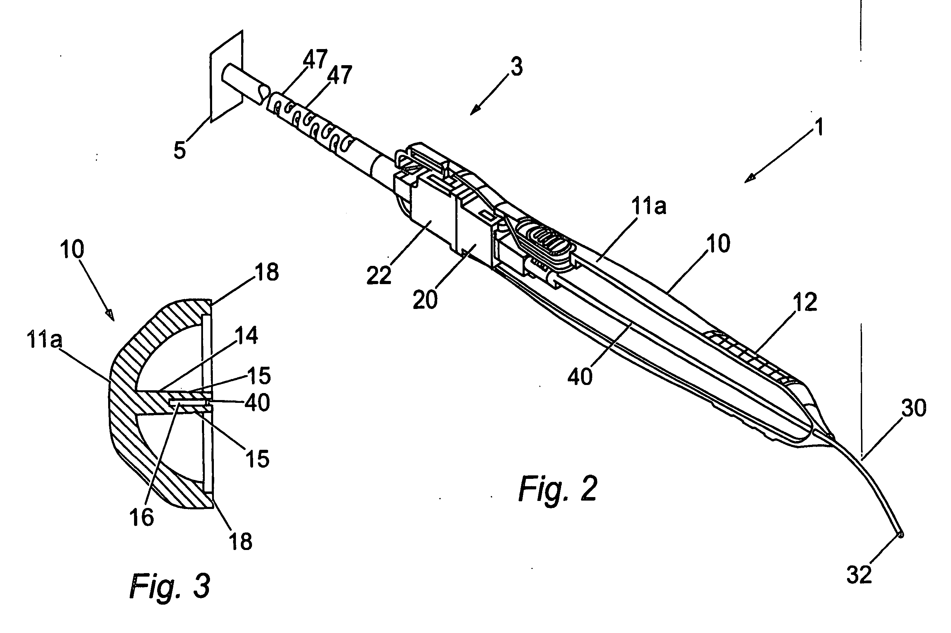

Referring to Figs. 1 and 2 there is shown a hand apparatus 1 according to an embodiment of the present invention, comprising a housing 10, a second optical connector 20 provided in the housing 10 and adapted for connection to a light source 3, an elongate sleeve 30 having a light emitter 32 at one end, and an optical carrier 40 extending from the optical connector 20 to the light emitter 32.

The second optical connector 20 is connected to a first optical connector 22 provided in a connector housing 24. Both connectors 20, 22 are typically standard optical telecommunication connectors such as those manufactured by Stratos Lightwave. Standard connectors allow accurate optical coupling of the fibre ends within a low tolerance (typically 1 micron).

The first optical connector 22 is typically a female connector and the second optical connector 20 is typically a male connector. This ensures that no optical carriers 40 are exposed while the first optical connector 22 is connected to the light source 3 but not connected to the second optical connector 20.

The optical carrier 40 comprises a number of optical fibres arranged in a bundle and generally surrounded by a sheath (not shown). The light source 3 includes a light generator 5 and an optical cable 45 comprising a number of optical fibres within a shielding connecting the light generator 5 to the first optical connector 22 in the connector housing 24. The cable 45 may include ribs or thickened portions 47 adjacent to the hand apparatus for increased flexural strength, flexural stiffness and fatigue life.

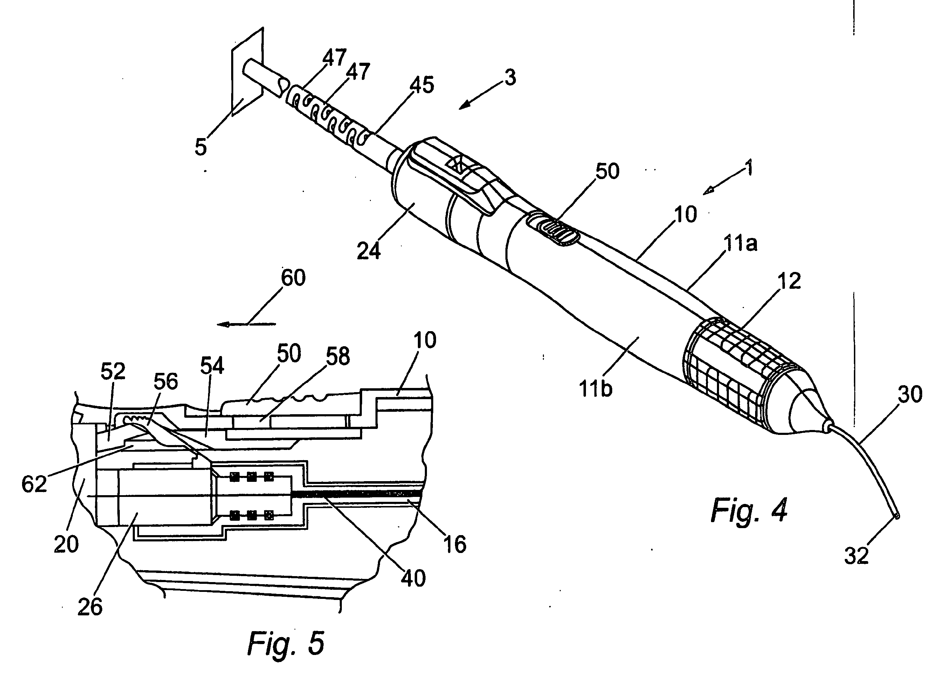

A switch 50 is provided on the housing 10. The switch may be seen most clearly in Fig. 5. The switch 50 is connected through a slot 58 to a cam member 54. When the switch 50 is operated in the direction of arrow 60, the cam member 54 is driven forward in the direction of arrow 60. This forward travel of the cam member 54 causes the hinged latch control member 56 to move downwardly and engage with a latch member 52 which also moves downwardly and causes the first optical connector 22 to decouple from the second optical connector 20. An abutment member 62 is also connected to the switch 50 and after a predetermined amount of forward movement, during which the optical connector 22 is decoupled as described above, abuts the first optical connector 22 causing the first and second optical connectors to separate. This separation provides a visible signal to the operator that the hand piece 1 can be removed from the light source 3. It is to be understood that the switch mechanism illustrated in Fig. 5 is only one possible switch mechanism and may be replaced by any suitable hand operated coupling and decoupling system.

A suitable safety means (not shown) may also be provided such that light may only be transmitted from the first optical coupler 22 if the first optical coupler 22 is connected to the second optical coupler 20. The safety means may be an electronic switch which is broken when the hand apparatus 1 is disconnected from the light source 3, causing an electronic signal to be sent along wires (not shown) in the cable 45 to turn off the light generator 5.

The housing 10 comprises two components 11a,11b joined to each other using snap-fit connection or other suitable means. The housing 10 is typically manufactured from moulded plastic. The housing is substantially cylindrical in shape.

Fig. 3 shows one component 11a of the housing in cross section (at III-III in Fig. 1) and also the optical carrier 40. The housing, at this axial location, includes a web portion 14 extending from the outer circumference towards the centre. The web portion 14 divides into two split web portions 15, which together with the two split web portions 15 of the other component 11a define a channel 16 in which the optical carrier 40 is supported in a way which allows transverse movement within the channel.

The ends 18 of the outer circumferential portion of the component 11a are profiled to allow snap-fit connection with correspondingly shaped ends 18 of the other component 11b. When the two components 11a, 11b of the housing 10 are joined, the split web portions 15 of the two components of the housing 10 form a channel which houses the optical carrier 40 and extends along the axis of the housing 10 from the second optical connector 20 to the sleeve 30.

Fig. 4 shows the hand apparatus with the two components 11a, 11b of the housing 10 joined. At one region of the exterior surface of the housing 10 there is provided a grip portion 12 having an embossed surface. This region is typically located near the sleeve end of the housing.

Referring also to Figs. 1 and 2, the sleeve 30 is fixed at one end within the housing 10. At the other end of the sleeve 30 is located the light emitter 32. The light emitter may be an isotip optical emitter of the sort described in United States Patent No. 5,073,402.

The internal diameter of the sleeve 30 is substantially greater than the external diameter of the optical carrier 40. The sleeve 30 is typically made of stainless steel or other suitable metal. This allows the sleeve 30 to be permanently bent into a position desirable to the user. Because the optical carrier 40 is free to move longitudinally relative to the sleeve 30, bending the sleeve 30 causes no damage to the optical carrier 40.

The optical carrier 40 is constrained where it is joined to the second optical connector 20 and is fixed at the light emitter 32 but is otherwise unconstrained, and is free to move laterally within the channel 16 and within the sleeve 30. The optical carrier 40 is rigidly secured to the second coupler 26, which is mounted within the second connector 20 in a resiliently biased manner, in order to ensure a firm connection between the second coupler 26 and the first coupler 28, which is fixedly mounted in the first connector 22. When the first 22 and second 20 optical connectors are connected, the second coupler 26 moves longitudinally relative to the second connector 20 as it is brought into contact and pressed against the first coupler 28. It is therefore important that the housing accommodates relative longitudinal movement of the optical carrier 40, which is fixed to the second coupler 26, and the housing 10, which is fixed to the second connector 20. This relative longitudinal movement is accommodated by the fact that the channel 16 in which the optical carrier 40 sits is much wider than the carrier itself. Hence the carrier 40 is free to adopt a serpentine profile in order to accommodate the longitudinal movement of the end attached to the second coupler 16. The dimensions of the channel 16 typically allow up to 2 mm relative longitudinal movement.

The hand apparatus 1 as described is inexpensive to produce. It is intended that the hand apparatus 1 be a disposable unit. The light source 3, including the first optical connector 22 and the connector housing 24 and the associated cabling 45 are intended to be more permanent. The hand apparatus 1 can readily be disconnected from the light source 3 by simple separation of the first and second optical connectors 20, 22 and replaced by another hand apparatus. If required different types of hand apparatus 1 having different lengths of housing 10 and/or different lengths and/or shapes of sleeve 30 may also be provided.

The hand apparatus 1 may also be used to provide other functions, particularly dental functions. For instance, a fluid conduit (not shown) may also be provided within the housing 10 that supplies fluid, such as mouth-rinse, to a spout (not shown) located adjacent to the light emitter 32.

Modifications and variations to the invention described above are possible without departing from the scope of the invention.

## Claims

1. A hand apparatus (1) comprising:
a housing; (10),
a light source (3);
an optimal connector (20, 22);
an elongate sleeve (30) having a light emitter (32) at one end, and an optical carrier (40) extending from the optical connector (20, 22) to the light emitter (32),
**characterised in that**:
the optical connector comprises a first optical connector (22) which is connected to the light source (3) via a number of optical fibres and a second optical connector (20) which is connectable to the first optical connector (22) and the optical carrier (40),
and that the second optical connector (20) is provided in the housing (10).

2. A hand apparatus (1) according to Claim 1, wherein the optical connector (20, 22) is a male or female optical connector adapted for connection to optical telecommunication fibres.

3. A hand apparatus (1) according to any preceding claim, wherein the optical connector (20, 22) includes a resiliently biased optical coupler (26) to ensure contact between the optical coupler (26) of the connector (20, 22) and the light source (3) to which it is to be connected.

4. A hand apparatus (1) according to any preceding claim, including safety means adapted to prevent light being transmitted if the optical connector (20, 22) is disconnected from the light source (3).

5. A hand apparatus (1) according to any preceding claim, including a switch (50) adapted to operate a detent member (52) which allows disconnection of the connector (20, 22) from the light source (3) upon operation of the switch (50).

6. A hand apparatus (1) according to any preceding claim, including retaining means (16) for retaining the optical carrier (40) within the housing (10), the retaining means (16) being adapted to permit longitudinal movement of the optical carrier (40) relative to the retaining means (16) during connection of the connector (20, 22) to a light source (3).

7. A hand apparatus (1) according to Claim 6, wherein the retaining means (16) comprises an internal channel extending from the connector (20, 22) to the sleeve (30), the optical carrier (40) extending along the channel.

8. A hand apparatus (1) according to any preceding claim, including a fluid delivery system comprising:
a reservoir;
a fluid outlet adjacent the light emitter (32); and
a conduit connecting the reservoir and the fluid outlet, the conduit allowing the passage of fluid from the fluid reservoir to the outlet adjacent the light emitter (32) via the housing (10) .

9. A hand apparatus (1) according to any preceding claim, wherein the sleeve (30) is adapted to permit longitudinal movement of the optical carrier (40) relative to the sleeve (30) upon deformation or flexing of the sleeve (30).

10. A hand apparatus (1) according to any preceding claim, wherein the sleeve (30) is resiliently flexible.

11. A hand apparatus (1) according to any of Claims 1 to 9, wherein the sleeve (30) is plastically deformable.

## Patentansprüche

1. Ein Handapparat (1), umfassend:
ein Gehäuse (10);
eine Lichtquelle (3);
einen optischen Steckverbinder (20, 22);
eine langgestreckte Hülse (30) mit einem Lichtaustritt (32) an einem Ende und einem Lichtleiter (40), der sich vom optischen Steckverbinder (20, 22) bis zum Lichtaustritt (32) erstreckt,
**dadurch gekennzeichnet, dass**:
der optische Steckverbinder einen ersten optischen Steckverbinder (22), der mit der Lichtquelle (3) über eine Anzahl optischer Fasern verbunden ist, und einen zweiten optischen Steckverbinder (20) umfasst, der mit dem ersten optischen Steckverbinder (22) und dem Lichtleiter (40) verbunden werden kann,
und dass sich der zweite optische Steckverbinder (20) in dem Gehäuse (10) befindet.

2. Ein Handapparat (1) nach Anspruch 1, wobei der optische Steckverbinder (20, 22) ein optischer Stecker oder eine optische Steckdose und geeignet für den Anschluss an optische Telekommunikationsfasern ist.

3. Ein Handapparat (1) nach einem der vorhergehenden Ansprüche, wobei der optische Steckverbinder (20, 22) einen elastisch vorgespannten optischen Koppler (26) zur Sicherung des Kontaktes zwischen dem optischen Koppler (26) des Steckverbinders (20, 22) und der mit ihm zu verbindenden Lichtquelle (3) enthält.

4. Ein Handapparat (1) nach einem der vorhergehenden Ansprüche, enthaltend eine Sicherungsvorrichtung, die geeignet ist, die Lichtaussendung zu verhindern, wenn der optische Steckverbinder (20, 22) von der Lichtquelle (3) abgekoppelt ist.

5. Ein Handapparat (1) nach einem der vorhergehenden Ansprüche, enthaltend einen Schalter (50), der geeignet ist, ein Klinkenteil (52) zu betätigen, welches bei Betätigung des Schalters (50).die Trennung des Steckverbinders (20, 22) von der Lichtquelle (3) ermöglicht

6. Ein Handapparat (1) nach einem der vorhergehenden Ansprüche, enthaltend ein Halterungsmittel (16) zur Halterung des Lichtleiters (40) innerhalb des Gehäuses (10), wobei das Halterungsmittel (16) so ausgelegt ist, dass es während des Verbindens des Steckverbinders (20, 22) mit einer Lichtquelle (3) eine Längsbewegung des Lichtleiters (40) relativ zum Halterungsmittel (16) zulässt.

7. Ein Handapparat (1) nach Anspruch 6, wobei das Halterungsmittel (16) einen inneren Kanal umfasst, welcher sich vom Steckverbinder (20, 22) bis zur Hülse (30) erstreckt, und wobei der Lichtleiter (40) in dem Kanal verläuft.

8. Ein Handapparat (1) nach einem der vorhergehenden Ansprüche, enthaltend eine Flüssigkeitsabgabeeinrichtung, die
einen Tank,
eine dem Lichtaustritt (32) benachbarte Flüssigkeitsauslassöffnung sowie
eine den Tank und die Flüssigkeitsauslassöffnung verbindende
Leitung
umfasst, wobei die Leitung das Fließen von Flüssigkeit vom Tank zu der dem Lichtaustritt (32) benachbarten Flüssigkeitsauslassöffnung durch das Gehäuse (10) ermöglicht.

9. Ein Handapparat (1) nach einem der vorhergehenden Ansprüche, wobei die Hülse (30) geeignet ist, bei einer Verformung oder Biegung der Hülse (30) eine Längsbewegung des Lichtleiters (40) relativ zur Hülse (30) zuzulassen.

10. Ein Handapparat (1) nach einem der vorhergehenden Ansprüche, wobei die Hülse (30) elastisch flexibel ist.

11. Ein Handapparat (1) nach einem der Ansprüche 1 bis 10, wobei die Hülse (30) plastisch verformbar ist.

## Revendications

1. Un appareil à main (1) comprenant :
un logement (10) ;
une source de lumière (3) ;
un connecteur optique (20, 22) ;
un manchon allongé (30) ayant un émetteur de lumière (32) au niveau d'une extrémité, et un support optique (40) s'étendant du connecteur optique (20, 22) à l'émetteur de lumière (32).
**caractérisé en ce que** :
le connecteur optique comprend un premier connecteur optique (22) qui est connecté à la source de lumière (3) par le biais d'un certain nombre de fibres optiques et un deuxième connecteur optique (20) qui peut être connecté au premier connecteur optique (22) et au support optique (40),
et **en ce que** le deuxième connecteur optique (20) est prévu dans le logement (10).

2. Un appareil à main (1) selon la revendication 1, dans lequel le connecteur optique (20, 22) est un connecteur optique mâle ou femelle adapté pour être connecté à des fibres optiques de télécommunication.

3. Un appareil à main (1) selon n'importe quelle revendication précédente, dans lequel le connecteur optique (20, 22) inclut un coupleur optique décalé de façon résiliente (26) pour assurer le contact entre le coupleur optique (26) du connecteur (20, 22) et la source de lumière (3) à laquelle il doit être connecté.

4. Un appareil à main (1) selon n'importe quelle revendication précédente, incluant des moyens de sécurité adaptés pour empêcher que de la lumière soit transmise si le connecteur optique (20, 22) est déconnecté de la source de lumière (3).

5. Un appareil à main (1) selon n'importe quelle revendication précédente, incluant un commutateur (50) adapté pour actionner un élément de détente (52) qui permet la déconnexion du connecteur (20, 22) de la source de lumière (3) lors de l'actionnement du commutateur (50).

6. Un appareil à main (1) selon n'importe quelle revendication précédente, incluant un moyen de retenue (16) destiné à retenir le support optique (40) au sein du logement (10), le moyen de retenue (16) étant adapté pour permettre le déplacement longitudinal du support optique (40) relativement au moyen de retenue (16) pendant la connexion du connecteur (20, 22) à une source de lumière (3).

7. Un appareil à main (1) selon la revendication 6, dans lequel le moyen de retenue (16) comprend un canal interne s'étendant du connecteur (20, 22) au manchon (30), le support optique (40) s'étendant le long du canal.

8. Un appareil à main (1) selon n'importe quelle revendication précédente, incluant un système d'apport de fluide comprenant :
un réservoir ;
une sortie de fluide adjacente à l'émetteur de lumière (32) ;
et
un conduit connectant le réservoir et la sortie de fluide, le conduit permettant le passage de fluide du réservoir de fluide à la sortie adjacente à l'émetteur de lumière (32) par le biais du logement (10).

9. Un appareil à main (1) selon n'importe quelle revendication précédente, dans lequel le manchon (30) est adapté pour permettre le déplacement longitudinal du support optique (40) relativement au manchon (30) lors de la déformation ou de la flexion du manchon (30).

10. Un appareil à main (1) selon n'importe quelle revendication précédente, dans lequel le manchon (30) est flexible de façon résiliente.

11. Un appareil à main (1) selon n'importe lesquelles des revendications 1 à 9, dans lequel le manchon (30) est déformable de façon plastique.
